# EUROPÄISCHE PATENTANMELDUNG

(11) **EP 3 431 658 A1**
(43) Veröffentlichungstag der Anmeldung: **23.01.2019**
(21) Anmeldenummer: 18000596.9
(22) Anmeldetag: 11.07.2018
(51) Int. Cl.: E01C 19/28

(54) **BODENVERDICHTER UND VERFAHREN ZUR BESTIMMUNG VON UNTERGRUNDEIGENSCHAFTEN MITTELS EINES BODENVERDICHTERS**

(30) Priorität: 18.07.2017 DE 102017006844
(71) Anmelder: BOMAG GmbH, 56154 Boppard (DE)
(72) Erfinder: Laugwitz, Niels, 56154 Boppard (DE)
(74) Vertreter: Heidler, Philipp

(57) **Zusammenfassung**

Bodenverdichter (1) mit einer beim Verdichten eines Untergrunds (U) um eine Rotationsachse rotierenden Bodenverdichtungsbandage (8), wobei die Bodenverdichtungsbandage (8) zum Bestimmen eines Steifigkeitsgrads des Untergrunds (U) einen Deformationssensor (11) zur Erfassung einer elastischen Deformation der Bodenverdichtungsbandage (8) aufweist.

## Beschreibung

Die Erfindung betrifft einen Bodenverdichter, insbesondere eine Walze, mit einer Bodenverdichtungsbandage, die beim Verdichten eines Untergrunds um eine Rotationsachse rotiert, und einer Auswerteeinrichtung. Ferner betrifft die Erfindung ein Verfahren zur Bestimmung eines Steifigkeitsgrads eines Untergrunds mittels eines Bodenverdichters, insbesondere mittels einer statischen oder oszillierenden Bodenverdichtungsbandage.

Gattungsgemäße Bodenverdichter sind zum Verdichten des Untergrunds vorgesehen und sind aus dem Stand der Technik beispielsweise in Gestalt von Straßenwalzen oder konkreter von Tandemwalzen, mit denen ein Bodenschichtaufbau und insbesondere eine Asphaltstraße inklusive ihres Unterbaus verdichtet werden kann, hinlänglich bekannt. Wesentliche Elemente eines Bodenverdichters, insbesondere eines selbstfahrenden Bodenverdichters, sind ein Maschinenrahmen, ein Antriebsmotor, eine Fahrerkabine sowie eine in Arbeitsrichtung gesehen vordere und hintere Bodenverdichtungsbandage. Zum Verdichten wird der Bodenverdichter in mehreren Arbeitsschritten über den zu verdichtenden Bodenschichtaufbau bewegt, wobei bei jeder Überfahrt eine weitere Verdichtung bis hin zu einer Maximalverdichtung erzielt wird. Nach Erreichen der Maximalverdichtung ist ein weiteres Verdichten des Untergrunds nicht mehr nötig oder gar kontraproduktiv, da es zu einer Auflockerung des verdichteten Bodenschichtsaufbaus oder zur Kornzertrümmerung führt. Aus diesem Grund ist es hilfreich, über den aktuellen Verdichtungsgrad des Untergrunds Kenntnis zu haben. Außerdem ist beim Einsatz von Bodenverdichtungswalzen die Bildung von Bugwellen von besonderer Bedeutung. Durch das mehr oder weniger starke Einsinken der Bandage in den Untergrund wird Material in Umfangsrichtung vor und hinter die Bandage verdrängt. Die Materialanhäufung vor der Bandage kann dabei so groß werden, dass sie aufgrund des Verschiebewiderstands nicht mehr vor der Bandage hergeschoben wird, sondern überrollt wird. Dies führt zu Fahrbahnunebenheiten.

Neben statischen Bodenverdichtern, bei denen ausschließlich ein Eigengewicht der Bodenverdichtungsbandage zur Verdichtung des Untergrunds führt, kennt der Stand der Technik darüber hinaus dynamische Bodenverdichter, bei denen zur Erhöhung der Verdichtungsleistung die Bodenverdichtungsbandage neben einer beim Überrollen des Untergrunds um eine Rotationsachse erfolgenden Rotationsbewegung eine zusätzliche Bewegung ausführt, um so im Untergrund eine Kornumlagerung in eine dichtere Schicht zu erwirken. Hierbei unterscheidet man zwischen vibrierenden und oszillierenden Bodenverdichtungsbandagen. Bei oszillierenden Bodenverdichtungsbandagen wird die Rotationsbewegung der Bodenverdichtungsbandage in Umlaufrichtung von einer Oszillationsbewegung überlagert, was dazu führt, dass eine Scherkraft von der Bodenverdichtungsbandage auf die Oberfläche des Untergrunds einwirkt. In der Regel wird dabei die Oszillationsbewegung durch zwei in der Bodenverdichtungsbandage zur Rotation angetriebenen Schwungmassen hervorgerufen. Bei den vibrierenden Bodenverdichtungsbandagen nimmt die Bodenverdichtungsbandage einen Vibrationszustand ein, in dem die Bodenverdichtungsbandage eine Vibrationsbewegung durchführt, wodurch die Bodenverdichtungsbandage mit einer auf den Untergrund gerichteten und wegen der Vibrationsbewegung zeitlich modulierten Kontaktkraft auf den Untergrund einwirkt. Wegen der zeitlichen Modulation der Kontaktkraft und der daraus resultierenden zeitlich veränderlichen Deformation des Untergrunds lässt sich die bei vibrierenden Bodenverdichtungsbandagen auftretende Kontaktkraftänderung zur Bestimmung des Steifigkeitsgrads des Untergrunds heranziehen, wie es beispielsweise aus der Druckschrift DE 10 2010 052 713 A1 bekannt ist, wobei der Steifigkeitsgrad Maß für die Verdichtung im Untergrund ist. Da es statischen Bodenverdichtern an einer derartigen zeitlich modulierten Kontaktkraft fehlt, lassen sich die Verfahren zur Bestimmung eines Steifigkeitsgrads nicht von den vibrierenden Bodenverdichtungsanlagen auf die statischen Bodenverdichter übertragen. Bei Bodenverdichtern mit oszillierender Bodenverdichtungsbandage treten zwar zeitlich veränderliche Schubkräfte auf, diese sind allerdings hauptsächlich von den Reibungsverhältnissen zwischen Bandage und Untergrund abhängig und eignen sich nur schlecht zur Ermittlung der Bodensteifigkeit.

Es ist deshalb eine Aufgabe der vorliegenden Erfindung, einen Bodenverdichter bereitzustellen, der geeignet ist, einen aktuellen Steifigkeitsgrad des Untergrunds und damit den aktuellen Bodenverdichtungsgrad zu bestimmen, ohne dass eine vibrierende Bodenverdichtungsbandage erforderlich ist. Insbesondere ist es eine Aufgabe der vorliegenden Erfindung, einen Bodenverdichter mit einer oszillierenden oder statischen Bodenverdichtungsbandage bereitzustellen, wobei der Bodenverdichter trotz der oszillierenden oder statischen Bodenverdichtungsbandage im Betrieb den Steifigkeitsgrad des Untergrunds erfassen kann. Außerdem ist es eine Aufgabe der vorliegenden Erfindung, Fahrbahnunebenheiten aufgrund von zu großen Bugwellen vor der Bandage zu verhindern.

Die Lösung der Aufgabe gelingt mit einem Bodenverdichter sowie einem Verfahren gemäß den unabhängigen Ansprüchen. Bevorzugte Weiterbildungen sind in den abhängigen Ansprüchen angegeben.

Konkret schlägt die Erfindung vor, einen Deformationssensor zur Erfassung einer elastischen Deformation an der Bodenverdichtungsbandage anzuordnen, um mittels der erfassten elastischen Deformation der Bodenverdichtungsbandage auf den Steifigkeitsgrad des Untergrunds zu schließen. Zur Auswertung der Messergebnisse weist der Bodenverdichter eine Auswerteeinrichtung auf, die beispielsweise als Recheneinheit und insbesondere als Bordcomputer ausgebildet ist beziehungsweise Teil des Bordcomputers des Bodenverdichters ist. Es ist nun erfindungsgemäß vorgesehen, dass die Bodenverdichtungsbandage einen Deformationssensor zur Erfassung einer elastischen Deformation der Bodenverdichtungsbandage aufweist, und dass die Auswerteeinrichtung dazu ausgebildet ist, den Steifigkeitsgrad des Untergrunds und/oder die Form einer in einer Arbeitsrichtung vor der Bodenverdichtungsbandage liegenden Bugwelle und/oder die Form einer Einsenkmulde der Bodenverdichtungsbandage aus der Lage eines Punktes auf einem Umfang der Bodenverdichtungsbandage, an dem der Deformationssensor die größte Deformation misst, zu bestimmen. Der Deformationssensor ist also insbesondere dazu ausgebildet, einen Maximalwert der Deformation der Bodenverdichtungsbandage zu ermitteln und dessen Lage an die Auswerteeinrichtung zu übermitteln. Die vom Deformationssensor übermittelte Lage kann sich dabei auf die Lage des Punktes maximaler Deformation innerhalb des Messbereiches des Deformationssensors oder die Lage im Bezug auf einen Rotationswinkel der Bodenverdichtungsbandage beziehen. Im ersten Fall wird die Lage in Bezug auf den Rotationswinkel wie nachstehend beschrieben durch eine zusätzliche Rotationswinkelbestimmung gemessen. Ein wesentlicher Aspekt hierbei ist, dass sich nicht nur der Untergrund, sondern auch die Bodenverdichtungsbandage durch einen Kontakt mit dem Untergrund, insbesondere elastisch, deformiert und dass sich diese Deformation abhängig von einem Kontaktzustand zwischen dem Bodenverdichtungsbandage und dem Untergrund ändert. Da der Kontaktzustand wiederum von der Steifigkeit des Untergrunds abhängt, lässt sich durch eine Überwachung der Bandagendeformation mittelbar Aufschluss gewinnen über den Steifigkeitsgrad des Untergrunds. Grundsätzlich ist der Begriff Kontaktzustand hier als Sammelbegriff für alle den Kontakt zwischen Untergrund und Bodenverdichtungsbandage betreffenden Faktoren zu verstehen. Dabei wird der Kontaktzustand beispielsweise durch einen Kontaktbereich, der sich zwischen dem Untergrund und der Bodenverdichtungsbandage durch deren Kontakt ausbildet, geprägt, wobei mit zunehmender Steifigkeit der Kontaktbereich kleiner wird, da die Bandage weniger in den Bodenuntergrund einsinkt. Hierbei verjüngt sich der Kontaktbereich mit zunehmender Steifigkeit insbesondere in einer durch die Umlaufrichtung der Bodenverdichtungsbandage vorgegebenen Richtung. Da diese den Kontaktzustand prägenden Einflüsse bei jeder Bodenverdichtungsbandage auftreten, lässt sich mit dem erfindungsgemäßen Bodenverdichter der Steifigkeitsgrad bestimmen, ohne dass eine vibrierende Bodenverdichtungsbandage erforderlich ist. Konkret sieht die Erfindung vor, denjenigen Punkt am Umfang der Bodenverdichtungsbandage zu finden, an dem die elastische Deformation der Bodenverdichtungsbandage während der Überfahrt über den Untergrund am größten ist. Die Lage dieses Punktes hängt vom Kontaktzustand beziehungsweise von der Form des Kontaktbereiches der Bodenverdichtungsbandage mit dem Bodenuntergrund ab. Es handelt sich um denjenigen Punkt, an dem die Deformation der Bodenverdichtungsbandage ein lokales Maximum erreicht. Der Winkel beziehungsweise Rotationswinkel der Bodenverdichtungsbandage, der die Lage dieses Punktes beschreibt, wird nachfolgend ebenfalls als kritischer Rotationswinkel bezeichnet. Der Erfindung liegt die Erkenntnis zugrunde, dass die Lage des Punktes der maximalen Deformation der Bodenverdichtungsbandage mit steigender Verdichtung des Untergrundes immer näher an den vertikal untersten Punkt der Bodenverdichtungsbandage heranrückt. Mit anderen Worten nähert sich der Punkt der maximalen Deformation der Bodenverdichtungsbandage mit zunehmender Steifigkeit des Untergrundes einem Punkt auf dem Umfang der Bodenverdichtungsbandage an, der vertikal unter der Rotationsachse der Bodenverdichtungsbandage liegt. Dieser Zusammenhang ermöglicht es, aus der Lage des Punktes auf den Kontaktzustand beziehungsweise die Form des Kontaktbereiches der Bodenverdichtungsbandage mit dem Untergrund zu schließen und damit einen Rückschluss auf die Steifigkeit des Untergrundes zu treffen. Die Bodensteifigkeit lässt sich dann als Indikator für eine erreichte Verdichtung heranziehen, d. h. es lässt sich anhand der erfassten Bodensteifigkeit auf einen Verdichtungszustand des Untergrunds schließen, beispielsweise unter Heranziehen eines E-Moduls, eines Schwingungsverhaltens und/oder einer Dichte des Materials, aus dem der Bodenbelag gefertigt ist. Hierbei ist es auch vorstellbar, dass zur Bestimmung des Verdichtungsgrads zusätzlich die Asphalttemperatur während des Verdichtungsprozesses mitberücksichtigt wird. Ferner ist es insbesondere vorgesehen, dass die elastische Deformation während des Betriebs des Bodenverdichters überwacht wird. Dadurch lässt sich der Steifigkeitsgrad bestimmen, ohne dass die Bodenverdichtung unterbrochen werden muss. Eine entsprechende Berechnung der Untergrundsteifigkeit kann rechnerisch aus den gemessenen Deformationen beziehungsweise der Lage des Punktes der maximalen Deformation erfolgen. Hierzu müsste allerdings eine exakte Kenntnis über die tatsächliche Bandagengeometrie inklusive der Fertigungstoleranzen vorhanden sein. Alternativ zur direkten Berechnung ist es daher ebenfalls vorgesehen, den Verdichtungsgrad anhand von Kennfeldern zu ermitteln, die für den entsprechenden Bodenverdichter erstellt wurden. Derartige Kennfelder werden also im Voraus erstellt und beispielsweise elektronisch im Bordcomputer des Bodenverdichters hinterlegt. Die Kennfelder beschreiben dabei insbesondere den Zusammenhang zwischen Bodensteifigkeit und der Lage des Punktes der maximalen Deformation. Aus der gemessenen Lage kann daher im Arbeitsbetrieb des Bodenverdichters aus den Kennfeldern die entsprechende Bodensteifigkeit beziehungsweise der Verdichtungsgrad des Bodens entnommen werden. Wie schon erwähnt kann zur Ermittlung der Lage des Punktes maximaler Deformation ein lokales Maximum der vom Deformationssensor ermittelten Messwerte herangezogen werden. Zeitlich und im Messbereich des Deformationssensors örtlich gesehen kommt es vor und hinter dem Punkt der maximalen Deformation aufgrund einer entgegengesetzten Deformation zu lokalen Minima der Messwerte des Deformationssensors. Alternativ zur Bestimmung des Maximums kann nun ebenfalls vorgesehen sein, die Position dieser lokalen Deformationsminima über den Deformationssensor zu ermitteln. Da der Punkt der maximalen Deformation näherungsweise in der Mitte der beiden Minima liegt, kann aus der Lage der Minima auf die Position des Punktes der maximalen Deformation geschlossen werden. Darüber hinaus ist die Kontaktzone der Bodenverdichtungsbandage mit dem Untergrund proportional zum Abstand der beiden Minima. Aus der Lage der beiden Minima kann daher die Breite der Kontaktzone abgeschätzt werden. Durch den Vergleich mit im Voraus festgelegten Kennfeldern kann aus der Breite der Kontaktzone ebenfalls ein Rückschluss auf die Untergrundsteifigkeit und/oder die Einsinktiefe der Bodenverdichtungsbandage gezogen werden. Alternativ zu den beschriebenen Minima könnte ebenfalls der Nulldurchgang der Deformation genutzt werden. Der Nulldurchgang entspricht denjenigen Punkten, an dem die vom Deformationssensor gemessene Deformation zwischen negativer und positiver Deformation wechselt. Derartige Punkte liegen ebenfalls im Wesentlichen symmetrisch vor und nach dem Maximum.

Alternativ oder ergänzend ist es vorgesehen, dass anhand der erfassten Lage des Punktes maximaler Deformation auf die Form, insbesondere Größe und/oder Ausdehnung, der Bugwelle in Arbeitsrichtung vor der Bodenverdichtungsbandage geschlossen wird. Die sogenannte Bugwelle ist ein weiterer den Kontaktzustand prägender Faktor. Hierbei handelt es sich um eine in Arbeitsrichtung gesehen unmittelbar vor der Bodenverdichtungsbandage zu findende Materialakkumulation, deren Größe mit zunehmender Steifigkeit des Untergrunds abnimmt. Diese Bugwelle verursacht wiederum einen Rollwiderstand, der den Deformationscharakter der Bodenverdichtungsbandage mitprägen kann. Mit abnehmender Größe dieser Bugwelle verliert der durch die Bugwelle verursachte Rollwiderstand an Einfluss auf den Kontaktzustand und damit auf die elastische Deformation der Bodenverdichtungsbandage. Infolgedessen wird der Einfluss der Bugwelle auf den Deformationscharakter fahrrichtungsunabhängig. Vorzugsweise wird der Fahrzeugführer über die aktuelle Form der Bugwelle informiert, so dass er bei Bedarf - z. B. bei einem Wechsel der Arbeitsrichtung - zur Vermeidung einer späteren Bodenunebenheit in Form der Bugwelle entsprechende Gegenmaßnahmen einleiten kann. Mögliche Maßnahmen sind der Einsatz einer weniger schweren Walze, Verringerung der angebotenen Verdichtungsenergie oder die Abkühlung des heißen Asphaltmischgutes zuzulassen, so dass sich dessen Verformungswiderstand erhöht. Wenn die Bugwelle klein genug ist, so dass die Gefahr der Bildung von Unebenheiten gering ist, kann die Verdichtungsleistung erhöht werden, z.B. durch Einsatz der Vibration. Es ist auch vorstellbar, dass anhand der Lage des Punktes maximaler Deformation auf eine Form, insbesondere Tiefe, einer Einsenkmulde und anschließend über die Einsenkmulde indirekt auf den Verdichtungsgrad geschlossen wird. Alternativ oder ergänzend ist es vorgesehen, dass durch die Bestimmung der zeitlichen Abstände zwischen den Deformationsmaxima die durchschnittliche Rotationsgeschwindigkeit der Bandage ermittelt wird. Somit kann beispielsweise die Breite der Deformationszone näherungsweise ermittelt werden, ohne dass der Rotationswinkel mit einem entsprechenden Sensor gemessen wird. Dabei wird eine konstante Fahrgeschwindigkeit vorausgesetzt, die sehr häufig im praktischen Einsatz gegeben ist. Bei plötzlichen starken Abweichungen der Periodendauer der Defomationsmaxima kann von einem Reversiervorgang ausgegangen werden und das Messergebnis wird verworfen. Da die Erfassung des Deformationsmaximums unter Umständen eine sehr hohe Abtastrate erfordert und zudem durch zufällige Störungen des Messsignals beeinträchtigt sein kann, kann zusätzlich die Steigung dε/dφ vor und hinter dem Maximum ermittelt werden. Bei niedrigen Untergrundsteifigkeiten ist der Gradient de/dφ geringer als bei hohen Untergrundsteifigkeiten. Analog kann die Ausdehnung der Kontaktzone durch den Abstand der lokalen Minima der Bandagendeformation charakterisiert werden.

Grundsätzlich ist unter einer Deformation der Bodenverdichtungsbandage jede Formänderung, die die Bodenverdichtungsbandage während der Rotationsbewegung um die Rotationsachse im Verdichtungsbetrieb erfährt, zu verstehen. Insbesondere wird unter der Deformation diejenige Formveränderung verstanden, die die Bodenverdichtungsbandage beim Überrollen des Untergrunds auf Grund des Kontakts zwischen Untergrund und Bodenverdichtungsbandage erfährt. Hierbei erfolgt die Formänderung vorzugsweise hauptsächlich am Umfang der Bodenverdichtungsbandage bzw. im Bandagenmantel. Eine typische Deformation kann beispielsweise eine Biegung, insbesondere eine lokale Dehnung oder Stauchung, der Bodenverdichtungsbandage sein.

Vorzugsweise umfasst die Bodenverdichtungsbandage einen im Wesentlichen hohlzylindrisch ausgebildeten Bandagenmantel mit einer der Rotationsachse zugewandten Innenseite, wobei der Deformationssensor an der Innenseite des Bandagenmantels angeordnet ist. Dadurch ist der Deformationssensor auf der dem Untergrund abgewandten Seite der Bodenverdichtungsbandage geschützt im Bereich, in dem die Deformation auftritt, angeordnet. Für einen schnellen Zugriff, beispielsweise zu Wartungszwecken, ist der Deformationssensor vorzugsweise in einem Endbereich der Bodenverdichtungsbandage weniger als 25 cm, bevorzugt weniger als 15 cm und besonders bevorzugt weniger als 10 cm von einer der Stirnseiten bzw. einer Außenkante der Bodenverdichtungsbandage entfernt angeordnet. Ein im Wesentlichen ungehinderter Zugriff auf den Deformationssensor erlaubt es den Deformationssensor bedarfsabhängig von der Bodenverdichtungsbandage zu demontieren oder an der Bodenverdichtungsbandage zu befestigen. Vorzugsweise umfasst der Bodenverdichter einen lösbaren Koppelmechanismus, mit dem der Deformationssensor mit wenigen Handgriffen von der Bodenverdichtungsbandage gelöst oder an dieser montiert werden kann, beispielsweise eine Schraubverbindung. Weiterhin ist es bevorzugt vorgesehen, wenn der Bodenverdichter eine in Arbeitsrichtung gesehen vordere und eine in Arbeitsrichtung gesehen hintere Bodenverdichtungsbandage umfasst, die jeweils einen Deformationssensor aufweisen. Es ist auch vorstellbar, dass der Deformationssensor an einer Tellerscheibe der Bandage angeordnet ist.

Zur Verbesserung der räumlichen Auflösung sind vorzugsweise mehrere Deformationssensoren vorgesehen. Mittels eines mehrere Deformationssensoren umfassenden Systems lässt sich in vorteilhafter Weise die Anzahl an Messpunkten erhöhen. So kann die Lage des Punktes der maximalen Deformation der Bodenverdichtungsbandage bei in Umfangsrichtung verteilten mehreren Deformationssensoren entsprechend öfter pro Umdrehung der Bodenverdichtungsbandage ermittelt werden. Auf diese Weise können Veränderungen der Bodensteifigkeit schneller detektiert werden. Ferner ist es denkbar, dass bei einer Verwendung von zwei Bandagen jeweils ein Deformationssensor an einer in Arbeitsrichtung gesehen vorderen und an einer in Arbeitsrichtung gesehen hinteren Bandage angeordnet ist, so dass eine durch die vordere Bandage verursachte Verdichtungszunahme mittel des Deformationssensors an der hinteren Bandage ermittelt werden kann. Vorzugsweise wird bei einer solchen Vorgehensweise ein Bewegungsprofil des Bodenverdichters, beispielsweise eine Bewegung im Hundegang, mitberücksichtigt.

Zur Erfassung der im Verdichtungsprozess auftretenden, insbesondere im Wesentlichen elastischen, Deformation der Bodenverdichtungsbandage genügt es einen lokalen Bereich der Bodenverdichtungsbandage, vorzugsweise kontinuierlich, in Hinblick auf seine Deformation, die dieser lokale Bereich während einer vollständigen Umdrehung der Bodenverdichtungsbandage erfährt, zu überwachen. Durch eine Anordnung des Deformationssensors innerhalb eines Teilbereichs der Bodenverdichtungsbandage wird der Deformationssensor während einer vollständigen Umdrehung zumindest einmal eine Deformation messen, die zur Bestimmung des Steifigkeitsgrads herangezogen werden kann. Erfindungsgemäß ist es vorgesehen, dass die Deformationsänderung innerhalb einer vollständigen Umdrehung in Abhängigkeit vom Rotationswinkel der Bodenverdichtungsbandage gemessen wird, wodurch die Lage des Punktes maximaler Deformation ermittelbar ist. Die Winkelstellung der Bodenverdichtungsbandage kann dabei mit sämtlichen im Stand der Technik bekannten Möglichkeiten ermittelt werden. Zu diesem Zweck umfasst der Bodenverdichter vorzugsweise eine Messeinrichtung, mit der der Rotationswinkel der Bodenverdichtungsbandage erfasst werden kann, insbesondere um die relative Lage des Deformationssensors zum Bodenuntergrund ermitteln zu können. Beispielsweise handelt es sich bei der Messeinrichtung zur Bestimmung des Rotationswinkels um einen Drehratensensor oder einen Beschleunigungsaufnehmer, insbesondere einen zweiachsigen Beschleunigungsaufnehmer. Auch vorstellbar ist, einen Impulsgeber zur Bestimmung des Rotationswinkels heranzuziehen. Hierbei stellt der Impulsgeber eine bestimmte Anzahl digitaler Impulse pro Umdrehung bereit, wodurch der Rotationswinkel durch ein Zählen der Impulse ermittelt wird. Eine besonders unkomplizierte Methodik zur Bestimmung des Rotationswinkels sieht es vor, dass bei einer gleichmäßigen Bewegung, d. h. einer Bewegung mit einer im Wesentlichen konstanten Geschwindigkeit, eine maximale Deformation in regelmäßigen Zeitabständen auftritt, so dass bei Verwendung eines einzelnen Deformationssensors anhand des Messausschlags und der Winkelgeschwindigkeit auf den aktuellen Rotationswinkel geschlossen werden kann. Grundsätzlich dient die Bestimmung des Rotationswinkels als Maß für die Ausrichtung der Bodenverdichtungsbandage bei deren Rotation, wobei die jeweilige Ausrichtung der Bodenverdichtungsbandage während einer vollständigen Umdrehung genau einem Rotationswinkel eindeutig zugeordnet ist. Beispielsweise ist der Rotationswinkel, insbesondere der Winkel 0°, durch die Position des Deformationssensors, festgelegt.

Grundsätzlich ist jeder Deformationssensor, mit dem eine elastische Deformation der Bodenverdichtungsbandage erfasst werden kann, im Rahmen der Erfindung einsetzbar. Denkbar wäre beispielsweise, dass innerhalb der Bodenverdichtungsbandage eine Querstrebe verläuft, auf die bei einer elastischen Deformation des Bodenverdichtungsbandage ein Druckkraft wirkt, die wiederum gemessen werden kann, oder dass ein speziell ausgelegtes Kamerasystem zur Bestimmung der elastischen Deformation herangezogen wird. In einer bevorzugten Ausführungsform wird ein Dehnungssensor in die Bodenverdichtungsbandage eingelassen, bzw. an der Innenseite des Bandagenmantels platziert. Beispielsweise handelt es sich bei dem Dehnungssensor um einen piezoelektrischen Dehnungssensor, bei dem eine auf ein piezoelektrisches Element wirkende Kraft, beispielsweise eine durch die Dehnung verursachte Zugkraft, eine Spannung verursacht, die erfasst und als Messgröße für die Dehnung der Bandage herangezogen werden kann. Grundsätzlich umfasst der Dehnungssensor insbesondere wenigstens einen Dehnungsmessstreifen, einen Dehnungstrafo, ein Faser-Bragg-Gitter etc. Die Verwendung eines solchen, insbesondere als Dehnungsmessstreifen ausgebildeten, Dehnungssensors hat den Vorteil, dass er vergleichsweise flach an der Innenseite des Bandagenmantels angebracht werden kann und entsprechend wenig Bauraum im Bodenverdichter bzw. in der Bodenverdichtungsbandage in Anspruch nimmt. Zudem lassen sich mit Dehnungssensoren besonders gut solche Dehnungen messtechnisch erfassen, die Resultat einer elastischen Deformation sind, die beim Überrollen des Untergrunds aufgrund des Kontakts zwischen Untergrund und Bodenverdichtungsbandage auftreten. Bei einer vollständigen Umdrehung wird der Dehnungssensor dann relevante Messergebnisse liefern, wenn der Dehnungssensor am Kontaktbereich während der Rotationsbewegung der Bodenverdichtungsbandage vorbeigeführt wird. Insbesondere ist es vorgesehen, dass die Dehnung entlang einer durch die Umlaufrichtung der Bodenverdichtungsbandage vorgegebenen Richtung überwacht wird. Dabei ist es vorstellbar, dass der Dehnungssensor für einen festgelegten Rotationswinkelbereich aktiviert wird und außerhalb des festgelegten Rotationswinkelbereichs in einen Stand-by-Betrieb übergeht.

Grundsätzlich kann die Lage des Punktes maximaler Deformation der Bodenverdichtungsbandage auf verschiedene Arten ermittelt werden. Eine besonders genaue Messung wird gemäß einer bevorzugten Ausführungsform erreicht, wenn die Auswerteeinrichtung dazu ausgebildet ist, einen Rotationswinkel, der von einer die Rotationsachse und den Punkt maximaler Deformation der Bodenverdichtungsbandage schneidenden Geraden und einer Referenzgeraden, beispielsweise einer horizontalen oder einer vertikalen Geraden, aufgespannt wird, zur Ermittlung des Steifigkeitsgrads des Untergrunds und/oder der Form der Bugwelle und/oder der Form der Einsinkmulde heranzuziehen. Insbesondere die Richtung des Gravitationsvektors lässt sich als vertikaler Referenzgerade besonders zuverlässig und einfach ermitteln, beispielsweise mittels eines zweiachsigen Beschleunigungssensors, und bietet sich daher als Referenzgerade zur Ermittlung der Lage des Punktes maximaler Deformation an. Die Lage des Punktes maximaler Deformation wird dann durch denjenigen Winkel ausgedrückt, der zwischen der vertikalen Referenzgeraden und einer Verbindungslinie zwischen der Rotationsachse der Bodenverdichtungsbandage und dem Punkt maximaler Deformation liegt. Dieser Winkel ist insbesondere proportional zur Tiefe der Einsinkmulde beziehungsweise zum Höhenunterschied des Bodenuntergrundes in Arbeitsrichtung vor und hinter der Bodenverdichtungsbandage, wie nachstehend noch näher erläutert wird.

Um einem Nutzer die gemessenen Deformationen und/oder die Lage des Punktes der maximalen Deformation und/oder die ermittelten Werte der Bodensteifigkeit beziehungsweise der Verdichtung und/oder der Form der Bugwelle und/oder der Einsenkmulde benutzerfreundlich zur Verfügung zu stellen, ist es erfindungsgemäß vorgesehen, dass der Bodenverdichter eine Auswerteeinrichtung umfasst. Mittels einer solchen Auswerteeinrichtung lässt sich beispielsweise die Abhängigkeit der vom Deformationssensor gemessenen Deformation vom Rotationswinkel auf einer Anzeigevorrichtung darstellen. Vorzugsweise ist die Anzeigevorrichtung im Fahrerstand angeordnet, so dass ein Nutzer bzw. Fahrzeugführer Kenntnis über den Steifigkeitsgrad des Untergrunds erhält, ohne den Fahrerstand verlassen zu müssen. Ferner ist es vorstellbar, dass der Bodenverdichter über eine Eingabeeinrichtung und einer Speichereinrichtung verfügt. Dadurch lassen sich vorzugsweise gemessene Deformationen, insbesondere abhängig vom Rotationswinkel, zusammen mit Kommentaren, die beispielsweise eine Information über eine Bodenzusammensetzung, eine Außentemperatur oder eine Besonderheit bei der Erfassung der elastischen Deformation betreffen, abspeichern. Infolgedessen lässt sich mittels der Speichereinrichtung eine Datenbank erstellen, die bei einer zukünftigen Bestimmung des Steifigkeitsgrads zur Einschätzung des aktuellen Steifigkeitsgrads herangezogen werden kann.

In einer besonders bevorzugten Ausführungsform handelt es sich bei dem Bodenverdichter um einen Bodenverdichter mit einer statischen oder oszillierenden Bodenverdichtungsbandage. Die oszillierende Bodenverdichtungsbandage umfasst vorzugsweise zwei zur Rotation antreibbare Schwungmassen, die innerhalb der Bodenverdichtungsbandage angeordnet sind. Diese zwei Schwungmassen rotieren gleichsinnig um zwei Unwuchtachsen, wobei die beiden Schwungmassen vorzugsweise in ihrer Massenverteilung einander entsprechen und im Betrieb phasenversetzt zueinander, insbesondere um 180° phasenversetzt zueinander, um ihre jeweilige Unwuchtachse rotieren. Hierbei sind die Unwuchtachsen paarweise gegenüberliegend angeordnet und die Rotationsachse der Bandage liegt mittig zwischen den Unwuchtachsen.

Gemäß einer weiteren Ausführungsform der vorliegenden Erfindung ist es vorgesehen, dass es sich bei dem Bodenverdichter um eine Tandemwalze handelt. Tandemwalzen zeichnen sich dadurch aus, dass sie eine vordere und eine hintere Bodenverdichtungsbandage aufweisen. Optimal ist es, wenn an wenigstens einer, insbesondere an beiden Bodenverdichtungsbandagen jeweils ein Deformationssensor in der vorstehend beschriebenen Weise vorhanden ist.

Die Lösung der Aufgabe gelingt ebenfalls mit einem Verfahren zur Bestimmung einer Steifigkeit des Untergrunds mittels eines Bodenverdichters gemäß den vorhergehenden Ausführungen. Sämtliche für den erfindungsgemäßen Bodenverdichter beschriebenen Merkmale und deren Vorteile lassen sich sinngemäß ebenfalls auf das erfindungsgemäße Verfahren übertragen und umgekehrt.

Konkret schlägt die Erfindung vor, dass in einem ersten Messschritt eine elastische Deformation zumindest in einem Teilbereich der Bodenverdichtungsbandage und die Lage eines Punktes auf einem Umfang der Bodenverdichtungsbandage, an dem die größte Deformation stattfindet, erfasst wird, wobei aus der Lage des Punktes der Steifigkeitsgrad des Untergrunds und/oder die Form einer in einer Arbeitsrichtung vor der Bodenverdichtungsbandage liegenden Bugwelle und/oder die Form einer Einsenkmulde der Bodenverdichtungsbandage bestimmt wird. Hierfür wird der Bodenverdichter mit um die Rotationsachsen rotierenden Bodenverdichtungsbandagen über einen zu verdichtenden Untergrund geführt. Die Erfassung der Lage des Punktes maximaler Deformation und damit die Bestimmung des Steifigkeitsgrads erfolgen dabei im Betrieb, d. h. während der Bodenverdichter den Untergrund überrollt. Insbesondere wird diejenige Deformation erfasst, die durch den Kontakt zwischen Bodenverdichtungsbandage und Untergrund hervorgerufen wird. Hierzu wird vorzugsweise die Deformation in der Nähe des Kontaktbereichs, der durch den Kontakt zwischen der Bodenverdichtungsbandage und dem Untergrund festgelegt wird, erfasst bzw. gemessen. Ferner ist es vorgesehen, dass die Deformation pro Umdrehung der Bodenverdichtungsbandage jeweils einmal erfasst wird, insbesondere beim Vorbeiführen des Deformationssensors am Kontaktbereich.

Zur Erfassung des Ausmaßes der elastischen Deformation in der Bodenverdichtungsbandage beziehungsweise der Lage des Punktes maximaler Deformation erweist sich eine Dehnungsmessung in einem lokalen Bereich der Bodenverdichtungsbandage als geeignet. Für die Messung der Dehnung ist ein Dehnungssensor vorgesehen, wobei sich hierbei zu Nutze gemacht wird, dass mit der elastischen Deformation eine Dehnung entlang einer durch die Umlaufrichtung vorgegebenen Richtung einhergeht, wobei sich die Dehnung auf einfache Weise mit dem Dehnungssensor erfassen lässt. Ein weiterer Vorteil ist, dass Dehnungssensoren vergleichsweise klein sind, so dass ein Dehnungssensor eine bauraumsparende Möglichkeit zur Erfassung der Deformation ist. Sofern der Dehnungssensor an der Innenseite des Bandagenmantels angeordnet ist, wird der Dehnungssensor bei einer vollständigen Umdrehung einmal vergleichsweise nahe am Kontaktbereich vorbeigeführt. Ferner ist es vorstellbar, dass der Dehnungssensor in einer Aussparung innerhalb der Bodenverdichtungsbandage angeordnet wird.

Erfindungsgemäß ist es vorgesehen, dass zur Bestimmung des Steifigkeitsgrads des Untergrunds die elastische Deformation in Abhängigkeit vom Rotationswinkel herangezogen wird. Der Rotationswinkel der Bodenverdichtungsbandage muss dabei nicht immer separat gemessen werden, da beispielsweise viele Fahrantriebe bereits über Vorrichtungen verfügen, die Aufschluss über den Rotationswinkel geben. Um die Messgenauigkeit zu erhöhen kann alternativ vorgesehen sein, dass neben der elastischen Deformation in einem zweiten Messschritt ein Rotationswinkel, beispielsweise mittels eines Drehratensensors oder einer Beschleunigungsaufnehmers, separat erfasst wird.

Zur Erhöhungen der Messgenauigkeit des Steifigkeitsgrads ist es insbesondere möglich, neben der beschriebenen Nutzung eines kritischen Rotationswinkel, in dem die gemessene Dehnung ein lokales Maximum annimmt, einen graduellen Verlauf der gemessenen Dehnung in Abhängigkeit des Rotationswinkels und/oder eine absolute Größe der gemessenen maximalen Dehnung heranzuziehen.

Nachfolgend wird die Erfindung anhand des in den Figuren angegebenen Ausführungsbeispiels näher erläutert. Es zeigen schematisch:
- Fig. 1: eine Seitenansicht auf einen Bodenverdichter in Gestalt einer Tandemwalze;
- Fig. 2: eine perspektivische Schrägansicht auf einen Bandagenmantel des Bodenverdichters aus Fig. 1;
- Fig. 3: in einem Flussdiagramm ein Verfahren zum Bestimmen eines Steifigkeitsgrads in einem Untergrund;
- Fig. 4a bis 4c: eine Abhängigkeit einer gemessenen Dehnung in einer Bodenverdichtungsbandage von dessen Rotationswinkel unter verschiedenen Bedingungen;
- Fig. 5: eine Seitenansicht einer Bodenverdichtungsbandage beim Überrollen eines Untergrundes niedriger Steifigkeit; und

- Fig. 6: eine Seitenansicht einer Bodenverdichtungsbandage beim Überrollen eines Untergrundes höherer Steifigkeit.

Gleiche Elemente sind in allen Figuren mit den gleichen Bezugszeichen versehen. Sich wiederholende Elemente sind zum Teil nicht in jeder Figur gesondert bezeichnet.

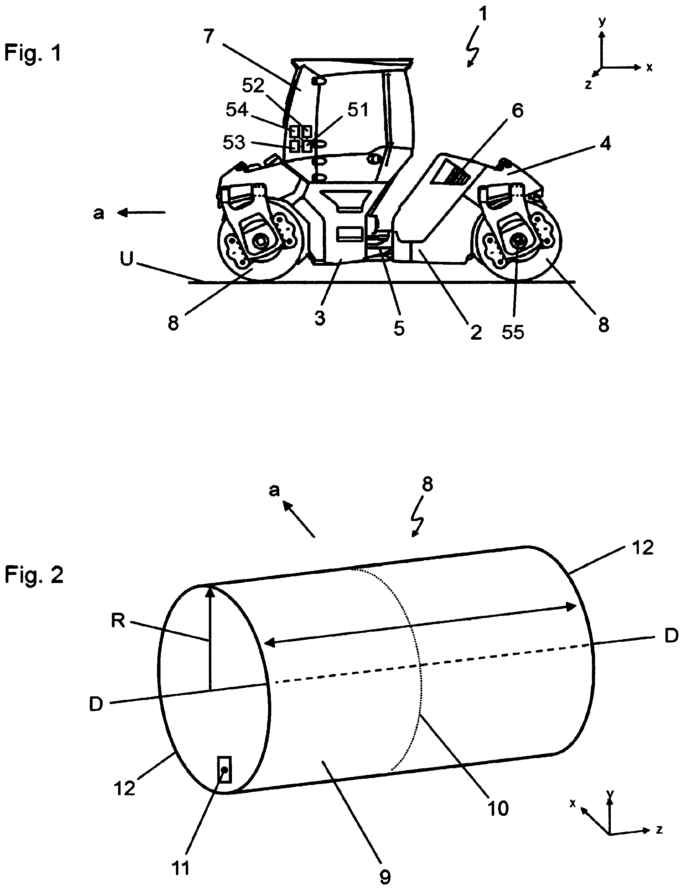

Die Figur 1 zeigt einen selbstfahrenden Bodenverdichter 1, bei dem es sich konkret um eine Tandemwalze handelt. Wesentliche Elemente der Walze sind ein Maschinenrahmen 2 mit einem Vorderrahmen 3 und einem Hinterrahmen 4, die über ein Knickgelenk 5 miteinander verbunden sind, ein Antriebsmotor 6, eine Fahrerkabine 7 sowie eine in Arbeitsrichtung a gesehen vordere und hintere Bodenverdichtungsbandage 8. Im Arbeitsbetrieb fährt die Walze 1 in oder entgegen der Arbeitsrichtung a über den zu verdichtenden Untergrund U und überrollt dabei mit den beiden Bodenverdichtungsbandagen 8 den Untergrund U. Es versteht sich, dass auch andere Bodenverdichter 1 in der nachfolgend beschriebenen Weise ausgebildet sein können, beispielsweise mit Drehschemellenkung etc.

Die Bodenverdichtungsbandagen 8 weisen einen im Wesentlichen hohlzylindrisch ausgebildeten Bandagenmantel 9 auf, wie er in Figur 2 schematisch wiedergegeben ist. Im Arbeitsbetrieb dreht die Bodenverdichtungsbandage 8 um ihre Drehachse D, die horizontal und quer zur Arbeitsrichtung a verläuft. Die Bodenverdichtungsbandage 8 bzw. der Bandagenmantel 9 weisen eine Breite B entlang der Achse D auf sowie einen Radius R. Der Bandagenmantel 9 kann einstückig oder zweiteilig mit zwei Bandagensegmenten ausgebildet sein, wie es in Figur 2 durch die gestrichelte Linie 10 angedeutet ist. Der Bandagenmantel 9 umgibt einen Bandageninnenraum, der in Radialrichtung durch den Bandagenmantel 9 begrenzt ist. In Richtung der Drehachse D erstreckt sich der Bandageninnenraum zu beiden Seiten jeweils bis zu den Stirnseiten bzw. in Radialrichtung zu den Außenkanten 12 des Bandagenmantels 9.

Ein wesentliches Aufgabengebiet der Walze besteht darin, einen Untergrund U, insbesondere einen Untergrund U einer Asphaltstraße, zu verdichten. Hierzu bewegt sich der Bodenverdichter 1 üblicherweise in mehreren Arbeitsschritten über den zu verdichtenden Untergrund U bis ein gewünschter Grad an Verdichtung bis hin zu einer Maximalverdichtung im Untergrund U erzielt wurde. Nach Erreichen der Maximalverdichtung ist ein weiteres Verdichten des Untergrunds U nicht mehr erforderlich oder gar kontraproduktiv, weil es zu einer erneuten Auflockerung des verdichteten Bodenschichtsaufbaus kommt. Insofern ist es hilfreich, im Betrieb über den aktuellen Grad der Verdichtung des Untergrunds U Kenntnis zu haben, um einen wirtschaftlichen und zuverlässigen Verdichtungsprozess zu ermöglichen.

Im Fall von Bodenverdichtern 1 mit einer Vibrationswalze wird zur Erhöhung der Verdichtungsleistung die Bodenverdichtungsbandage 8 in einen Vibrationszustand versetzt, in dem die Bodenverdichtungsbandage 8 eine Vibrationsbewegung durchführt. Dadurch wirkt die Bodenverdichtungsbandage 8 wiederum mit einer Kontaktkraft, die in ihrer Größe entsprechend der Vibrationsbewegung zeitlich moduliert ist, auf den Untergrund U ein. Dabei erfolgt das Einwirken in einem Kontaktbereich zwischen Bodenverdichtungsbandage B und Untergrund U entlang einer im Wesentlichen vertikalen, d. h. einer senkrecht zu Oberflächenkontur des Untergrunds U verlaufenden, Richtung. Hervorgerufen wird der Vibrationszustand der Bodenverdichtungsbandage 8 hierbei durch innerhalb der Bodenverdichtungsbandage 8 im Betrieb in im Stand der Technik bekannter Weise zur Rotation angetriebene Schwungmassen, die gezielt für das Erzeugen einer Unwucht und des gewünschten Vibrationszustands ausgelegt sind.

Im Falle eines Kreisrüttlers erfolgt dabei die Vibration mittels einer um die Rotationsachse R der Bodenverdichtungsbandage 8 rotierende Schwungmasse, während im Falle eines Gegenrüttlers zwei gegensinnig rotierende Schwungmassen die Bodenverdichtungsbandage 8 in den Vibrationszustand versetzen. Für solche Vibrationswalzen lässt sich die senkrecht auf den Untergrund U einwirkende und dabei zeitlich modulierte Kontaktkraft zur Bestimmung eines Steifigkeitsgrads, der als Maß für die Verdichtungsgrad des Untergrunds U herangezogen werden kann, nutzen.

Im Gegensatz zu den vorstehend beschriebenen Bodenverdichtern 1 mit Vibrationswalzen mangelt es vorliegenden Bodenverdichtern 1 mit einer statischen oder einer oszillierenden Bodenverdichtungsbandage 8 an einer zeitlich modulierten und dabei vertikal auf den Untergrund U gerichteten Kontaktkraft, die zur Bestimmung der Untergrundsteifigkeit herangezogen werden könnte. Bei Bodenverdichtern 1 mit einer statischen Bodenverdichtungsbandage 8 erfolgt hierbei die Verdichtung ausschließlich aufgrund des statischen Eigengewichts der Bodenverdichtungsbandage 8. Im Falle einer oszillierenden Bodenverdichtungsbandage 8 wird die eigentliche Rotationsbewegung der Bodenverdichtungsbandage 8 um die Rotationsachse R überlagert von einer oszillierenden Bewegung, durch die Scherkräfte auf den Untergrund U übertragen werden, die die Verdichtungsleistung erhöhen. Die die Rotationsbewegung überlagernde Oszillationsbewegung wird beispielweise durch zwei Schwungmassen erzeugt, wobei eine erste Schwungmasse entlang einer vertikal verlaufenden Richtung über und eine zweite Schwungmasse entlang der vertikal verlaufenden Richtung unter der Rotationsachse R angeordnet ist. Im Betrieb werden die erste Schwungmasse und die zweite Schwungmasse zu gleichsinnigen Rotationen innerhalb der Bodenverdichtungsbandage 8 angetrieben. Gegenüber den Vibrationswalzen haben oszillierende Bodenverdichtungsbandagen den Vorteil, dass die Bodenverdichter 8 weniger Erschütterungen im Umfeld der Maschine verursachen. Dadurch können Bauwerksschäden an umliegenden Gebäuden oder auf Brücken vermieden werden.

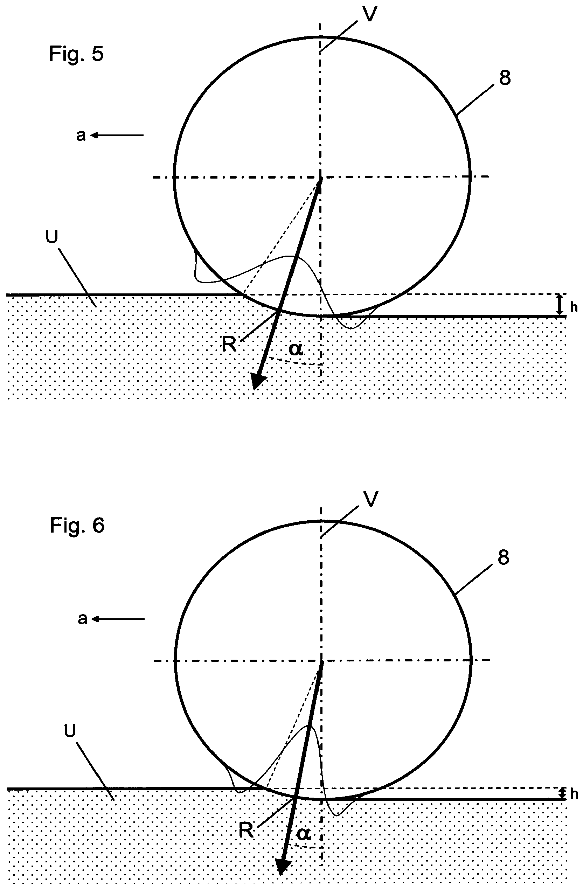

Um die Bestimmung des Steifigkeitsgrads des Untergrunds U auch bei dem Einsatz von Bodenverdichtern 1 mit statischer oder oszillierender Bodenverdichtungsbandage 8 zu ermöglichen, ist es erfindungsgemäß vorgesehen, dass während des Betriebs eine elastische Deformation der Bodenverdichtungsbandage 8 festgestellt wird. Hierzu umfasst die Bodenverdichtungsbandage 8 einen Deformationssensor 11. Anhand der Lage des Punktes R (siehe Figuren 5 und 6) maximaler gemessener Deformation auf dem Mantel der Bodenverdichtungsbandage 8, insbesondere in Kombination mit der Änderung der gemessenen Deformation und/oder der Deformationsänderung in Abhängigkeit von einem Rotationswinkel 101 der Bodenverdichtungsbandage, wird anschließend auf den Steifigkeitsgrad des Untergrunds U rückgeschlossen. Ein solcher Rückschluss auf den Steifigkeitsgrad des Untergrunds U ist insofern möglich, als dass sich eine Änderung eines Kontaktzustands zwischen dem Untergrund U und der Bodenverdichtungsbandage 8 in einer Änderung der elastischen Deformation der Bodenverdichtungsbandage 8 und damit auf die Lage des Punktes R maximaler gemessener Deformation niederschlägt. Wegen des sich abhängig vom Steifigkeitsgrad des Untergrunds U ändernden Kontaktzustands zwischen dem Untergrund U und der Bodenverdichtungsbandage 8 lässt sich daher in vorteilhafter Weise über die Lage des Punktes R mittelbar Rückschluss auf den Steifigkeitsgrad des Untergrunds U gewinnen. Die erfindungsgemäße Nutzung der Lage des Punktes R maximaler Deformation sorgt für eine besonders präzise Messung der Bodeneigenschaften.

Zu den Änderungen des Kontaktzustands, die sich in einer Änderung der elastischen Deformation niederschlagen, zählt beispielsweise eine mit zunehmender Bodenfestigkeit festzustellende Reduktion des Kontaktbereichs, der sich zwischen dem Untergrund U und der Bodenverdichtungsbandage 8 durch deren Kontakt ausbildet. Hierbei stellt man fest, dass diese Kontaktbereichsreduktion entlang einer durch die Umfangsrichtung der Bodenverdichtungsbandage 8 vorgegebenen Richtung erfolgt. Folge der Kontaktbereichsreduktion ist eine Zunahme eines maximalen Kontaktdrucks, der auf den Untergrund U einwirkt, wodurch letztendlich auch die elastische Deformation der Bodenverdichtungsbandage 8 beeinflusst wird. Außerdem wird der für die elastische Deformation relevante Kontaktzustand durch eine Bugwelle, die sich in Arbeitsrichtung a vor der Bodenverdichtungsbandage 8 ausbildet, beeinflusst. Diese Bugwelle wird mit zunehmender Bodensteifigkeit kleiner, wodurch eine Abhängigkeit des Kontaktzustands und damit der erfassbaren Deformation in Hinblick auf eine Fahrtrichtung, mit der der Bodenverdichter über den Untergrund U geführt wird, reduziert wird. All diese Faktoren wirken sich auf die Lage des Punktes R maximaler gemessener Deformation auf der Bodenverdichtungsbandage 8 aus, weshalb dieser als Referenz herangezogen werden kann.

Zur Erfassung der elastischen Deformation in der Bodenverdichtungsbandage 8 ist ein Deformationssensor 11 vorgesehen, der vorzugsweise ein Dehnungssensor ist. Mittels des Dehnungssensors lässt sich vorteilhaft in einem lokalen Bereich der Bodenverdichtungsbandage 8 eine elastische Deformation feststellen. Hierzu ist der Dehnungssensor 11 beispielsweise an einer Innenseite des Bandagenmantels 9 angeordnet. Um den Zugriff auf den Dehnungssensor 11, z.B. zum Auswechseln oder zur Instandsetzung, zu vereinfachen, ist der Dehnungssensor 11 vorzugsweise im Bereich einer der Stirnseiten bzw. der Außenkante 12 des Bandagenmantels 9 angeordnet. Weiterhin ist es bevorzugt vorgesehen, dass der Dehnungssensor 11 derart angeordnet ist, dass der Dehnungssensor 11 eine Dehnung in der Bodenverdichtungsbandage 8 entlang der Umlaufrichtung feststellt. Der Dehnungssensor 11 ermittelt denjenigen Punkt R, insbesondere bezogen auf den Umfang der Bodenverdichtungsbandage 8, an dem die Deformation der Bodenverdichtungsbandage 8 maximal ist. Dies kann beispielsweise in Bezug auf den Montageort des Deformationssensors 11 an der Bodenverdichtungsbandage 8 geschehen. Der Dehnungssensor 11 ermittelt den Punkt R innerhalb seines Messbereiches am Umfang der Bodenverdichtungsbandage 8. Der Rotationswinkel (α), in dem sich der Punkt R auf dem Umfang der Bodenverdichtungsbandage 8 befindet, kann aus dieser Messung und einer Information über den Rotationszustand beziehungsweise Rotationswinkel der Bodenverdichtungsbandage 8 ermittelt werden. Der Rotationszustand beziehungsweise Rotationswinkel der Bodenverdichtungsbandage 8 kann beispielsweise über die Verwendung von Fahrantrieben bekannt sein, die derartige Informationen bereitstellen. Insbesondere umfasst der Bodenverdichter 1 neben dem Deformationssensor 11 eine Messeinrichtung 55 zur Bestimmung des Rotationswinkels 101. Eine solche Messeinrichtung 55 erlaubt es, die gemessenen Deformationswerte, insbesondere die gemessenen Dehnungswerte 102, in Abhängigkeit vom Rotationswinkel 101 darzustellen und insbesondere die Lage des Punktes R maximaler Deformation auf dem Mantel der Bodenverdichtungsbandage 8 zu bestimmen. Unter dem Rotationswinkel 101 wird hierbei ein Maß für die Ausrichtung der Bodenverdichtungsbandage 8 bei seiner Rotation um die Rotationsachse R verstanden, wobei insbesondere jede mögliche Ausrichtung der Bodenverdichtungsbandage 8 einem bestimmtem Rotationswinkel 101 zwischen 0 und 360° zugeordnet werden kann. Vorzugsweise wird dabei die Zuordnung der Rotationswinkel 101 so vorgenommen, dass derjenigen Ausrichtung der Bodenverdichtungsbandage 8 der Winkel 0° zugeordnet wird, bei der der Deformationssensor 11, insbesondere der Dehnungssensor, dem Untergrund U am nächsten ist.

Ferner ist es vorstellbar, dass der Deformationssensor 11 eine Kommunikationseinrichtung, vorzugsweise eine Kommunikationseinrichtung zur drahtlosen Kommunikation, umfasst, mit der die erfassten Messwerte, die die Dehnung der Bodenverdichtungsbandage 8 repräsentieren, zu einer Auswerteeinrichtung 51 übermittelt werden. In der Auswerteeinrichtung 51 werden die aufgenommenen Messwerte analysiert und einem Nutzer, beispielsweise auf einer Anzeigeeinrichtung 52, bereitgestellt. Weiterhin ist es denkbar, dass der Bodenverdichter 1 eine Speichereinrichtung 54 umfasst. Eine solche Speichereinrichtung 54 erlaubt es die gemessenen bzw. analysierten Messwerte abzuspeichern und auf diese Weise ein Portfolio aus Erfahrungswerten bestehenden aus gemessen Deformationen 102, beispielsweise für verschiedene Untergrundtypen und/oder Asphalttemperaturen, zu erstellen. Mittels einer Eingabeeinrichtung 53 lassen sich zusätzlich Kommentare, die beispielsweise den Untergrundtyp, die Asphalttemperatur und/oder Besonderheiten betreffen, abspeichern.

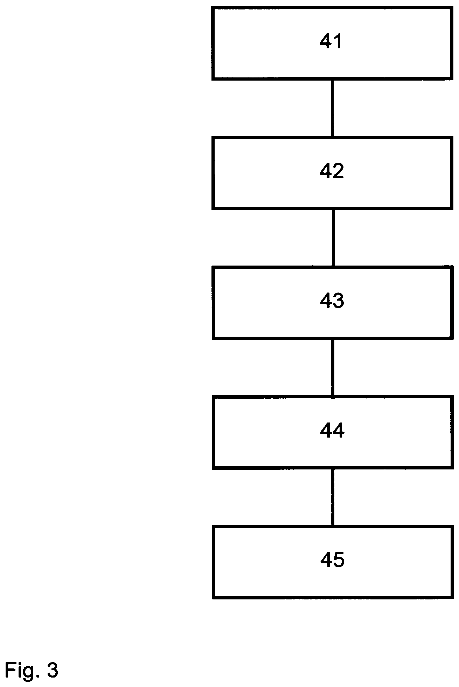

Anhand des in Figur 3 schematisch dargestellten Flussdiagramms soll im Folgenden die Vorgehensweise zur Bestimmung des Steifigkeitsgrads mittels eines Bodenverdichters 1, insbesondere eines Bodenverdichters, wie er in Figur 1 dargestellt ist, erläutert werden: Wesentliche Schritte des Verfahrens sind das Bewegen 41 eines Bodenverdichters 1 mit einer Bodenverdichtungsbandage 8 über den Untergrund U und das Rotieren 42 der Bodenverdichtungsbandage 8 um eine Rotationsachse R. Mittels eines Deformationssensors 11 zur Erfassung einer elastischen Deformation der Bodenverdichtungsbandage 8 wird in einem ersten Messschritt 43 die Deformation der Bodenverdichtungsbandage 8 überwacht. Hierbei ist es insbesondere vorgesehen, dass die Deformation kontinuierlich, d. h. im Wesentlichen ohne Unterbrechungen, erfasst bzw. gemessen wird. Sofern mit einem Dehnungssensor ein lokaler Bereich der Bodenverdichtungsbandage 11 in Hinblick auf eine mögliche Dehnung beobachtet wird, wird der Dehnungssensor während einer vollständigen Umdrehung der Bodenverdichtungsbandage 8 um die Rotationsachse R einmal, insbesondere innerhalb eines Intervalls der Rotation, eine signifikante Dehnungsänderung registrieren. Aus diesen Werten ermittelt der Deformationssensors 11 oder die Auswerteeinrichtung 51 die Lage des Punktes R maximaler Deformation. Mittels eines im zweiten Messschritt 43 aufgenommen Rotationswinkels 101 ist es möglich, die gemessenen Deformationen in Abhängigkeit vom aktuellen Rotationswinkel 101 in einem Auswerteschritt 45 darzustellen. Der Rotationswinkel 101 kann dabei entweder separat gemessen oder beispielsweise von einem Fahrantrieb bereitgestellt sein. Anhand der Abhängigkeit der gemessenen Deformation vom Rotationswinkel 101 ist es möglich, Änderungen in Hinblick auf das Deformationsverhalten der Bodenverdichtungsbandage 8 zu registrieren und die Lage des Punktes R maximaler Deformation auch im Hinblick auf einen Rotationswinkel der Bodenverdichtungsbandage 8 zu ermitteln. Hierbei ist es vorstellbar, dass dem Nutzer des Bodenverdichters 1 die Abhängigkeit der gemessenen Dehnung 102 graphisch dargestellt wird und der Nutzer auf Grundlage seines Erfahrungsschatzes und seiner Kenntnisse über den aktuellen Verdichtungsprozess, beispielsweise in Hinblick auf die Bodenbeschaffenheit, Rückschluss auf den Steifigkeitsgrad des Untergrunds U nimmt. Alternativ ist es auch vorstellbar, dass die Auswerteeinrichtung 51 die gemessene Abhängigkeit der elastischen Deformation vom Rotationswinkel 101 mit Vergleichsdaten aus einer Datenbank vergleicht und dem Nutzer im Anschluss an die Auswertung eine Kenngröße als Maß für den Steifigkeitsgrad bereitstellt. Es ist auch vorstellbar, dass die Auswerteeinrichtung ein Warnsignal, beispielsweise ein akustisches und/oder optisches Warnsignal ausgibt, wenn der Auswerteschritt 45 als Ergebnis den gewünschten Steifigkeitsgrad des Untergrunds U bzw. eine Zielbodenfestigkeit liefert.

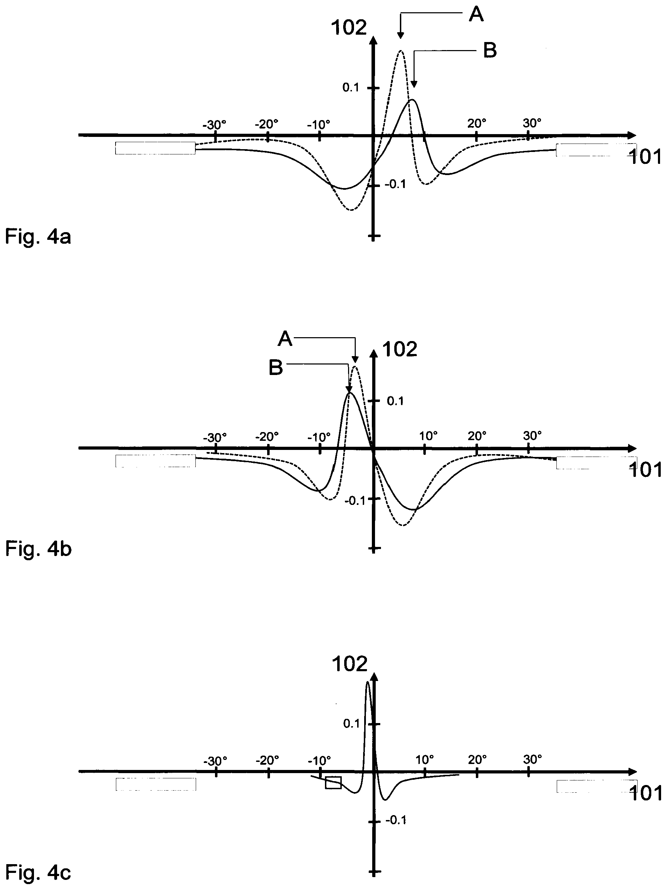

Die Figuren 4a bis 4c zeigen den prinzipiellen Verlauf von gemessenen Dehnungen 102 in Abhängigkeit des Rotationswinkels 101 unter verschiedenen Bedingungen. Diese prinzipiellen Verläufe wurden als Ergebnisse einer Versuchsreihe erhalten, bei der die Dehnung in einer Bodenverdichtungsbandage 8 gemessen wurde. Hierzu wurde eine von der Anmelderin vertriebene knickbare Tandemwalze vom Typ BW138 über den zu verdichtenden Untergrund U geführt. Diese Walze wurde im Vorfeld zur Bestimmung des Rotationswinkels 101 mit zwei zweiachsigen Beschleunigungsaufnehmern vom Typ AD22284 versehen. Zudem wurde an die Innenseite der in Arbeitsrichtung a gesehen hinteren Bodenverdichtungsbandage 8 mittels einer Schraube ein Dehnungssensor vom Typ CST/300 angeschraubt, der die hier als prinzipielle Verläufe schematisch dargestellten gemessenen Dehnungen 102 bereitstellte.

Die Figur 4a zeigt die gemessenen Deformationen 102 in willkürlicher Einheit in Abhängigkeit des Rotationswinkels 101, als die Walze mehrfach über ein Asphaltersatzmaterial geführt wurde. Dabei repräsentiert die durchgezogene Linie das gemessene Deformationsverhalten bei einem erstmaligen Überrollen des Asphaltersatzmaterials, während die gepunktete Linie das gemessene Deformationsverhalten bei einem erneuten Überrollen des Asphaltersatzmaterials mit einer erhöhten Bodenfestigkeit widerspiegelt. Hierbei bewegte sich die Walze jeweils in Arbeitsrichtung a. Charakteristisch für die beiden gemessenen und hier dargestellten Deformationsverläufe ist ein kritischer Rotationswinkel A, B, bei dem der jeweilige Verlauf ein Maximum einnimmt. Der Figur 4a entnimmt man, dass sich der kritische Rotationswinkel A bei einem erneuten Überrollen und damit bei einem höheren Steifigkeitsgrad gegenüber dem kritischen Rotationswinkel B beim erstmaligen Überrollen verschiebt. Außerdem lässt sich feststellen, dass ein absoluter Wert für die maximale gemessene Deformation 102, d. h. eine Amplitude für die gemessene Deformation 102, beim erneuten Überrollen gegenüber dem des erstmaligen Überrollens zugenommen hat.

Die Figur 4b illustriert die gemessenen Deformationen 102 in willkürlicher Einheit in Abhängigkeit des Rotationswinkels 101, als die Walze mehrfach über das Asphaltersatzmaterial geführt wurde. Der Versuch unterscheidet sich zunächst einmal von dem, der Figur 4a zugrunde liegt, dadurch, dass die Walze hier entgegen der Arbeitsrichtung a, also rückwärts, über den Untergrund U bewegt wurde. Beide Verläufe entstanden jeweils durch ein Überrollen, das sich an das erstmalige Überrollen anschließt, wobei ein Steifigkeitsgrad des Untergrunds U für die gepunktete Linie größer war als der für die durchgezogene Linie. Zusammen mit den Ergebnissen aus Figur 4a lässt sich auf Grundlage der Ergebnisse aus Figur 4b darauf schließen, dass mit zunehmender Bodenfestigkeit die Amplitude für die maximal gemessene Dehnung 102 zunimmt und der Abstand zwischen den kritischen Rotationswinkeln A, B, die sich jeweils bei aufeinanderfolgenden Überrollvorgängen ergeben, mit zunehmender Bodenfestigkeit abnimmt.

In der Figur 4c ist die gemessene Deformation in willkürlichen Einheiten in Abhängigkeit vom Rotationswinkel 101 dargestellt, als die Walze über ein festes, d.h. nicht mehr verdichtbares, Asphaltmaterial geführt wurde. Im Vergleich zu den Verläufen aus Figur 4a und 4b ist die Amplitude für die gemessene maximale Deformation genau wie eine graduelle Änderung der Deformation mit dem Rotationswinkel 101 im Bereich des kritischen Rotationswinkels A, B im Fall des festen Asphaltmaterials größer.

Als Ergebnisse der aus den Figuren 4a bis 4c beobachteten Verläufe lässt sich festhalten, dass anhand eines kritischen Rotationswinkels A, B, in dem die gemessene Dehnung 102 ein lokales Maximum annimmt, anhand eines graduellen Verlaufs der gemessenen Dehnung 102 in Abhängigkeit des Rotationswinkels 101 und/oder einer absoluten Größe der gemessenen maximalen Dehnung der Steifigkeitsgrad des Untergrunds U bestimmt bzw. rückgeschlossen werden kann.

In den Figuren 5 und 6 ist der Überrollvorgang eines Untergrundes U durch eine Bodenverdichtungsbandage 8 bei niedriger Steifigkeit des Untergrundes U (Figur 5) und bei höherer Steifigkeit des Untergrundes U (Figur 6) dargestellt. Zur Vereinfachung wird von ideal plastischem Untergrundverhalten ausgegangen, was sich aber in der Praxis als ausreichende Näherung herausgestellt hat. Die Bodenverdichtungsbandage 8 bewegt sich dabei in Arbeitsrichtung a von rechts nach links über den Untergrund U. Aufgrund der unterschiedlichen Steifigkeit des Untergrundes U nimmt die Einsinktiefe h mit der Zahl der Überrollungen ab. Die Einsinktiefe h wird auch als Setzungsmaß bezeichnet. Insbesondere geht aus den Figuren 5 und 6 die Ermittlung des Punktes R maximaler Deformation am Umfang der Bodenverdichtungsbandage 8 sowie der Zusammenhang der Lage des Punktes R mit der Steifigkeit des Untergrundes U beziehungsweise der Einsinktiefe h hervor. Zur Erläuterung sind in den Figuren die zeitlichen Verläufe der Deformation, gemessen durch den Deformationssensor 11, wie sie schon in den Figuren 4a bis 4c dargestellt wurden, durch die geschwungenen Linien angedeutet. Die gestrichelte Linie von der Rotationsachse der Bodenverdichtungsbandage 8 zeigt den vorderen Rand der Deformationsmulde an. Der Punkt R der maximalen Deformation liegt näherungsweise auf der Winkelhalbierenden zwischen dieser gestrichelten Linie und der vertikalen Vergleichsgeraden V. Insgesamt ermittelt der Deformationssensor 11, an welchem Punkt R des Umfanges der Bodenverdichtungsbandage 8 die Deformation maximal ist. Im dargestellten Ausführungsbeispiel wird dieser Punkt R durch den Rotationswinkel α der Bodenverdichtungsbandage 8, gemessen in Relation zu einer vertikalen Vergleichsgeraden V, beispielsweise der Richtung des Gravitationsvektors, angegeben. Selbstverständlich kann der Rotationswinkel α ebenfalls in Bezug auf eine andere Vergleichsgerade, beispielsweise eine horizontale Gerade, angegeben werden. Wichtig ist lediglich, dass die Lage des Punktes R am Umfang der Bodenverdichtungsbandage 8 ermittelt wird. Aus einem Vergleich der Figuren 5 und 6 ergibt sich, dass der Rotationswinkel α zwischen einer Verbindung der Rotationsachse der Bodenverdichtungsbandage 8 und dem Punkt R am Umfang der Bodenverdichtungsbandage 8 und der vertikalen Vergleichsgeraden V bei zunehmender Steifigkeit des Untergrundes U kleiner wird. Der Rotationswinkel α ist daher proportional zur Einsinktiefe h. Da sich der Punkt R dem Lot von der Rotationsachse der Bodenverdichtungsbandage 8 auf den Untergrund U annähert, kann aus der Lage des Punktes R ein Rückschluss auf die Steifigkeit des Untergrundes U beziehungsweise den Kontaktzustand der Bodenverdichtungsbandage 8 mit dem Untergrund U gezogen werden. Die Eindringtiefe h ergibt sich beispielsweise direkt aus dem Winkel α und dem Bandagenradius r nach folgender Formel: h = r . (1 - cos(2 . a)); Bei einem Bandagenradius r von beispielsweise 500 mm wäre ab einem Winkel α von < 1,25° das Setzungsmaß beziehungsweise die Einsinktiefe h kleiner als 0,5 mm. In diesem Stadium bei so geringer Eindringtiefe h müssten unverhältnismäßig viele Überfahrten durchgeführt werden, um noch eine messbare Verdichtungszunahme zu erreichen. Es ist daher das Verdichtungsziel erreicht. Neben der Erkennung des Verdichtungsendes durch die Feststellung, dass die gemessenen Winkel α sehr klein werden, kann über die vorliegende Erfindung ebenfalls festgestellt werden, wenn die Bugwelle vor der Bodenverdichtungsbandage 8 zu groß wird. Dies wird durch große Winkel α angegeben. Auf diese Weise kann der Bediener des Bodenverdichters rechtzeitig Gegenmaßnahmen einleiten, bevor die Bugwelle so groß wird, dass die Bodenverdichtungsbandage 8 sie überrollt und es so zu einer Fahrbahnunebenheit kommt. Ebenso wie für die Ermittlung der Bodensteifigkeit beziehungsweise des Verdichtungsgrades des Bodens aus der Lage des Punktes R der maximalen Deformation ist es auch für die Erkennung einer zu großen Bugwelle bevorzugt, im Voraus festgelegte Kennfelder für den Winkel α zur Auswertung zu nutzen. Die Auswertung erfolgt daher über empirisch ermittelte Grenzwerte, die zur Erstellung der Kennfelder genutzt werden.

## Patentansprüche

1. Bodenverdichter (1) mit einer beim Verdichten eines Untergrunds (U) um eine Rotationsachse (D) rotierenden Bodenverdichtungsbandage (8) und einer Auswerteeinrichtung (51),
**dadurch gekennzeichnet,**
**dass** die Bodenverdichtungsbandage (8) einen Deformationssensor (11) zur Erfassung einer elastischen Deformation der Bodenverdichtungsbandage (8) aufweist, und dass die Auswerteeinrichtung (51) dazu ausgebildet ist, den Steifigkeitsgrad des Untergrunds (U) und/oder die Form einer in einer Arbeitsrichtung (a) vor der Bodenverdichtungsbandage (8) liegenden Bugwelle und/oder die Form einer Einsenkmulde der Bodenverdichtungsbandage (8) aus der Lage eines Punktes (R) auf einem Umfang der Bodenverdichtungsbandage (8), an dem der Deformationssensor (11) die größte Deformation misst, zu bestimmen.

2. Bodenverdichter (1) gemäß Anspruch 1,
**dadurch gekennzeichnet,**
**dass** die Bodenverdichtungsbandage (8) einen im Wesentlichen hohlzylindrisch ausgebildeten Bandagenmantel (9) mit einer der Rotationsachse (D) zugewandten Innenseite umfasst, wobei der Deformationssensor (11) an der Innenseite des Bandagenmantels (9) angeordnet ist.

3. Bodenverdichter (1) gemäß einem der vorhergehenden Ansprüche,
**dadurch gekennzeichnet,**
**dass** der Deformationssensor (11) derart gestaltet ist, dass er in einem Teilbereich der Bodenverdichtungsbandage (8) die elastische Deformation erfasst.

4. Bodenverdichter (1) gemäß einem der vorhergehenden Ansprüche,
**dadurch gekennzeichnet,**
**dass** der Deformationssensor (11) ein Dehnungssensor zur Messung einer lokalen Dehnung in der Bodenverdichtungsbandage (8), insbesondere zur Messung einer in Umlaufrichtung erfolgenden lokalen Dehnung in der Bodenverdichtungsbandage (8), ist.

5. Bodenverdichter (1) gemäß einem der vorhergehenden Ansprüche,
**dadurch gekennzeichnet,**
**dass** der Bodenverdichter (1) eine Messeinrichtung (55) zur Erfassung eines aktuellen Rotationswinkels der Bodenverdichtungsbandage (8) aufweist.

6. Bodenverdichter (1) gemäß einem der vorhergehenden Ansprüche,
**dadurch gekennzeichnet,**
**dass** die Auswerteeinrichtung (51) dazu ausgebildet ist, einen Rotationswinkel (a), der von einer die Rotationsachse (D) und den Punkt (R) schneidenden Geraden und einer Referenzgeraden, beispielsweise einer horizontalen oder einer vertikalen Geraden, aufgespannt wird, zur Ermittlung des Steifigkeitsgrads des Untergrunds (U) und/oder der Form der Bugwelle und/oder der Form der Einsenkmulde heranzuziehen.

7. Bodenverdichter (1) gemäß einem der vorhergehenden Ansprüche,
**dadurch gekennzeichnet,**
**dass** der Bodenverdichter (1) zumindest eine der folgenden Einrichtungen aufweist:
- eine Anzeigeeinrichtung (52) zur Darstellung von mittels der Auswerteeinrichtung ermittelten Ergebnissen und/oder von mittels des Deformationssensor (11) erfassten Messwerten, insbesondere in Abhängigkeit des Rotationswinkels (101) und/oder
- einer Eingabeeinrichtung (53) zur Eingabe von Kommentaren und/oder
- eine Speichereinrichtung (54) zur Abspeicherung der Messergebisse und/oder der Kommentare.

8. Bodenverdichter (1) gemäß einem der vorhergehenden Ansprüche,
**dadurch gekennzeichnet,**
**dass** der Bodenverdichter (1) eine oszillierende oder eine statische Bodenverdichtungsbandage (8) aufweist.

9. Bodenverdichter (1) gemäß einem der vorhergehenden Ansprüche, wobei eine in Arbeitsrichtung (a) gesehen vordere und eine in Arbeitsrichtung (a) gesehen hintere Bodenverdichtungsbandage (8) jeweils zumindest einen Deformationssensor (11) aufweisen.

10. Bodenverdichter (2) gemäß einem der vorhergehenden Ansprüche,
**dadurch gekennzeichnet,**
**dass** der Bodenverdichter (1) eine Tandemwalze ist.

11. Verfahren zur Bestimmung eines Steifigkeitsgrads eines Untergrunds (U) mittels eines Bodenverdichters (1), insbesondere eines Bodenverdichters (1) gemäß einem der vorhergehenden Ansprüche,
umfassend die folgenden Schritte:
- Bewegen (41) eines Bodenverdichters (1) mit einer Bodenverdichtungsbandage (8) über den Untergrund (U) und
- Rotieren (42) der Bodenverdichtungsbandage (8) um eine Rotationsachse (D),
**dadurch gekennzeichnet,**
**dass** in einem ersten Messschritt (43) eine elastische Deformation zumindest in einem Teilbereich der Bodenverdichtungsbandage (8) und die Lage eines Punktes (R) auf einem Umfang der Bodenverdichtungsbandage (8), an dem die größte Deformation stattfindet, erfasst wird, wobei aus der Lage des Punktes (R) der Steifigkeitsgrad des Untergrunds (U) und/oder die Form einer in einer Arbeitsrichtung (a) vor der Bodenverdichtungsbandage (8) liegenden Bugwelle und/oder die Form einer Einsenkmulde der Bodenverdichtungsbandage (8) bestimmt wird.

12. Verfahren gemäß Anspruch 10,
**dadurch gekennzeichnet,**
**dass** im ersten Messschritt (43) als Maß für die elastische Deformation der Bodenverdichtungsbandage (8) eine lokale Dehnung der Bodenverdichtungsbandage (8), insbesondere eine in Umlaufrichtung der Bodenverdichtungsbandage erfolgenden lokale Dehnung der Bodenverdichtungsbandage, gemessen wird.

13. Verfahren gemäß Anspruch 10 oder 11,
**dadurch gekennzeichnet,**
**dass** in einem zweiten Messschritt (44) ein Rotationswinkel (101) der Bodenverdichtungsbandage (8) ermittelt und in einem Auswerteschritt (45) die elastische Deformation in der Bodenverdichtungsbandage (8) in Abhängigkeit des Rotationswinkels (101) dargestellt wird.

14. Verfahren gemäß Anspruch 12,
**dadurch gekennzeichnet,**
**dass** zusätzlich anhand
- eines graduellen Verlaufs der gemessenen Dehnung (102) in Abhängigkeit des Rotationswinkels (101) und/oder
- einer absoluten Größe der gemessenen maximalen Dehnung der Steifigkeitsgrad des Untergrunds (U) und/oder die Form der Bugwelle und/oder die Form der Einsenkmulde bestimmt wird.
